# EUROPÄISCHE PATENTSCHRIFT

(11) **EP 4 433 009 B1**
(45) Veröffentlichungstag und Bekanntmachung des Hinweises auf die Patenterteilung: **12.08.2026**
(21) Anmeldenummer: 23833105.2
(22) Anmeldetag: 18.12.2023
(51) Int. Cl.: A61F 13/00, A61F 13/01, A61F 13/02

(54) **ROLLE EINER MEDIZINISCHEN BANDAGE**
ROLL OF A MEDICAL BANDAGE
ROULEAU D'UN BANDAGE MÉDICAL

(30) Priorität: 22.12.2022 DE 102022134649
(43) Veröffentlichungstag der Anmeldung: 25.09.2024
(73) Patentinhaber: KOB GmbH, 67752 Wolfstein (DE)
(72) Erfinder: MERKT, Thomas, 67752 Wolfstein (DE)
(74) Vertreter: Paul Hartmann AG Patents & Licensing
(86) Internationale Anmeldenummer: PCT/EP2023/086253
(87) Internationale Veröffentlichungsnummer: WO 2024/133006

(56) Entgegenhaltungen:
- EP-A1- 3 298 998
- EP-B1- 2 257 254
- US-A- 5 061 258

## Beschreibung

Die Erfindung betrifft eine Rolle einer medizinischen Bandage zum Anlegen an einen menschlichen oder tierischen Körper sowie ein Herstellungsverfahren für eine solche Rolle.

Üblicherweise weisen Bandagen oder Verbände mit kohäsiven oder adhäsiven Eigenschaften, die als Rolle angeboten werden, einen starren zentralen Kern auf, der ein Kollabieren des inneren letzten Stücks des Verbandes auf der Rolle verhindert. Der zentrale Kern wird auch oft als Hülse bezeichnet. Ohne einen solchen starren Kern würde der letzte Abschnitt des Verbandes auf sich selbst zu kleben kommen und könnte nur noch sehr mühsam auseinandergezogen und anschließend angelegt werden. Zudem erleichtert die Hülse das Abrollen der Bandage, da der Anwender mit einem Finger der einen Hand in der Hülse die Rolle festhalten und mit der anderen Hand dann die Bandage abrollen kann.

Der starre zentrale Kern besteht in der Regel aus Pappe oder Kunststoff und stellt durch seine Steifigkeit auch die runde Form der Bandage für den Transport sicher.

Nachteilig ist jedoch, dass die Bereitstellung der Hülse beträchtlich zu den Gesamtkosten der Bandagenrolle beiträgt. Nicht nur die Herstellungskosten für die Hülse sind hoch, sondern verursacht die Hülse auch höhere Kosten für Logistik, da das Gewicht der Rolle durch die Hülse erhöht wird.

Auch aus Sicht der Nachhaltigkeit wäre es wünschenswert, auf die Hülse, die einen großen Materialbedarf aufweist und somit eine große Verschwendung an Kunststoff oder Pappe verursacht, zu verzichten. Eine weitere Einschränkung der derzeit bekannten Rollen ist die Umweltbelastung im Zusammenhang mit der Entsorgung der zentralen Kerne.

Aus der US 6,405,969 B1 ist ein Herstellungsverfahren für kernlose Rollen aus einem adhäsivem Bandmaterial bekannt. Das Bandmaterial ist einseitig mit einem Adhäsiv beschichtet, wobei in einem Endbereich das Adhäsiv mit einem Liner abgedeckt ist. Nach dem Aufrollen kommt der Liner im Inneren, also im Bereich eines axialen hohlen Durchlasses zu liegen und kleidet den axialen hohlen Durchlass entlang einer vollständigen Windung aus.

Die EP 3 298 998 A1 offenbart eine Rolle mit medizinischen Blättern aus Papiermaterial, die rund um ein zentrales Loch aufgewickelt sind. Zur Herstellung dieser Rolle wird das Papiermaterial auf einen elastischen Kern gewickelt, der anschließend durch hohe Geschwindigkeit wieder aus der Rolle entfernt wird. Eine solche hülsenlose Rolle vermag zwar das Gewicht der Rolle zu reduzieren und durch Weglassen der Hülse die Umwelt weniger zu belasten, jedoch ist das Verfahren nicht für selbstklebende oder kohäsive Bandagen anwendbar. Kernlose Rollen von selbstklebenden und kohäsiven Bandagen würden kollabieren und der letzte Abschnitt der Bandage auf sich selbst zu kleben kommen und die einzelnen aufeinander klebenden Lagen könnten nur noch sehr mühsam auseinandergezogen werden.

Aufgabe der vorliegenden Erfindung ist es, eine Rolle einer medizinischen Bandage bereitzustellen, die einfach und wirtschaftlich herzustellen ist und die Umweltbelastung reduziert.

Zudem liegt die Aufgabe zugrunde, ein Herstellungsverfahren für solch eine Rolle einer medizinischen Bandage vorzuschlagen.

Diese Aufgaben werden erfindungsgemäß durch eine Rolle nach Anspruch 1 und durch ein Verfahren nach Anspruch 10 gelöst.

Erfindungsgemäß ist eine Rolle einer medizinischen Bandage zum Anlegen an einen menschlichen oder tierischen Körper vorgesehen.

Die Rolle umfasst ein mit einer Vielzahl an Windungen spiralförmig gewickeltes flächiges und kohäsiv ausgestaltetes Bandmaterial. Das Bandmaterial weist an einem freistehenden Ende einen Endabschnitt auf und ist derart in einer Längsrichtung gewickelt, dass die Rolle mit einem axialen hohlen Durchlass gebildet ist. Der Endabschnitt des Bandmaterials liegt im Inneren der Rolle und somit am axialen hohlen Durchlass. Die Längsrichtung verläuft hierbei von dem einen freistehenden Ende des flächigen Bandmaterials zum anderen. Das flächige Material ist also von dem einen freistehenden Ende zum anderen freistehenden Ende aufgerollt, wobei der Endabschnitt die inneren Windungen darstellt. Der axiale hohle Durchlass ist in Bezug auf die Rolle mittig angeordnet und erstreckt sich entlang einer Querrichtung des Bandmaterials. Die Querrichtung verläuft im Wesentlichen rechtwinklig zu der Längsrichtung und verläuft üblicherweise parallel zur Schmalseite des Bandmaterials.

Erfindungsgemäß ist ein versteifender Einsatz aus einem flächigen Material mit einem vorderen Ende und einem hinteren Ende vorgesehen. Der versteifende Einsatz liegt im Endabschnitt auf dem Bandmaterial auf, insbesondere mit seiner gesamten Fläche, und umgibt wenigstens entlang einer vollständigen Windung den axialen hohlen Durchlass.

Der Term "mit seiner gesamten Fläche aufliegen" ist hierbei nicht absolut auszulegen, er soll vielmehr so verstanden werden, dass der versteifende Einsatz nahezu bzw. im Wesentlichen vollständig flächig auf dem Bandmaterial aufliegt. Sollte der versteifende Einsatz geringfügig über den Endabschnitt oder über das Bandmaterial hinausragen oder sich durch den gewickelten Zustand etwas von dem Bandmaterial abheben, so ist definitionsgemäß ein Aufliegen mit der gesamten Fläche weiterhin gegeben.

Unter dem Begriff "Windung" wird erfindungsgemäß der nahezu vollständige Verlauf des Bandmaterials um die zentrale Achse der Rolle verstanden. Somit liegt eine Windung vor, wenn beispielsweise das Bandmaterial oder der versteifende Einsatz einmal nahezu vollständig, also 360°, um den axialen hohlen Durchlass verläuft.

Die erfindungsgemäße Rolle ermöglicht die Einsparung einer stabilen Hülse im Bereich des axialen hohlen Durchlasses. Dadurch, dass der versteifende Einsatz entlang einer vollständigen Windung den axialen hohlen Durchlass umgibt, wird die Bandage im Endbereich stabilisiert und ein Kollabieren der Rolle effizient verhindert. Auf die Bereitstellung einer starren Hülse kann verzichtet werden. Anstelle der starren Hülse ist der versteifende Einsatz im Endbereich in die Rolle eingewickelt. Das Einwickeln kann in einen bestehenden Herstellungsprozesse integriert werden. Auch die Fertigungsanlagen müssen nur geringfügig angepasst werden. Dies führt zu einer erhebliche Kostenreduktion.

Da auch auf die starre Hülse, die üblicherweise ein höheres Flächengewicht als der versteifende Einsatz aufweist, verzichtet werden kann, wird das Gesamtgewicht der Rolle reduziert, was wiederum zu einer Kostenersparnis in der Logistik führt.

Somit reduziert die erfindungsgemäße Rolle auch die Umweltbelastung, da einerseits weniger Material für versteifenden Einsatz als für eine starre Hülse eingesetzt werden muss und andererseits weniger Energie verbraucht wird, sowohl durch das geringere Gewicht und der dadurch geringere Treibstoffverbrauch beim Transport als auch die Einsparung der Energie bei der Herstellung der starren Hülse.

Gemäß einer bevorzugten Ausführungsform ist vorgesehen, dass der versteifende Einsatz flexibel ausgebildet ist. Ein im Endabschnitt angeordneter flexibler versteifender Einsatz ermöglicht ein einfaches Aufwickeln des Bandmaterials zu einer Rolle. Gleichzeitig wird ein Kollabieren der Rolle verhindert.

Der versteifende Einsatz weist eine Länge auf, die so gewählt ist, dass sich das vordere Ende und das hintere Ende des versteifenden Einsatzes einander überlappen. Dabei ist nicht zwingend vorgesehen, dass das vordere Ende und das hintere Ende in direktem Kontakt miteinander stehen, also sich berühren. Ein Überlappen ist erfindungsgemäß auch dann gegeben, wenn zwischen dem vorderen Ende und dem hinteren Ende ein oder mehrere Lagen des Bandmaterials oder des versteifenden Einsatzes liegen.

Erfindungsgemäß ist die Länge des versteifenden Einsatzes derart eingerichtet, dass sich der versteifende Einsatz in der Längsrichtung über mehrere Windungen selbst überlappt, um einen spiralförmig ausgebildeten Versteifungsbereich zu bilden. Somit kann über die Länge des versteifenden Einsatzes die Steifigkeit des Versteifungsbereichs bestimmt werden. Je länger der versteifende Einsatz ist, desto öfters überlappt er sich selbst. Von der Mitte der Rolle, also dem axialen hohlen Durchlass aus gesehen, besteht die Rolle immer abwechselnd aus einer Windung des versteifenden Einsatzes und des Bandmaterials. Nach einer vorbestimmten Anzahl an Windungen ist das hintere Ende des versteifenden Einsatzes erreicht und jede Windung besteht nachfolgend aus Bandmaterial.

Der versteifende Einsatz kann in seiner Länge so gewählt sein, dass er den axialen hohlen Durchlass entlang von 1 bis 10 Windungen, vorzugsweise 1 bis 6 und besonders bevorzugt 2 bis 4 Windungen umgibt. Dies ermöglicht eine individuelle Steifigkeit des Versteifungsbereichs, je nach verwendetem Bandmaterial.

Besonders bevorzugt kann der versteifende Einsatz derart angeordnet sein, dass er den axialen hohlen Durchlass auskleidet. Somit besteht die innere Windung, die den axialen hohlen Durchlass bildet, aus dem versteifenden Einsatz.

Gemäß einer nicht erfindungsgemäßen Ausgestaltung kann die Länge des versteifenden Einsatzes derart eingerichtet sein, dass das vordere Ende und das hintere Ende mit jeweils ihrer Stirnseite aneinanderstoßen, jedoch ohne miteinander fest bzw. einstückig verbunden zu sein, wie dies bei einer Hülse der Fall wäre. Diese Ausführungsform weist den Vorteil auf, dass nur sehr wenig Material für den versteifenden Einsatz aufgewendet werden muss, da der versteifende Einsatz den axialen hohlen Durchlass lediglich entlang einer Windung umgibt.

Erfindungsgemäß ist der versteifende Einsatz derart angeordnet, dass er wenigstens entlang zweier Windungen den axialen hohlen Durchlass umgibt. Der versteifende Einsatz überlappt lediglich mit seiner äußeren Windung, nämlich der Windung, die am weitesten von der Mittelachse der Rolle entfernt liegt, das Bandmaterial. Somit stellt der versteifende Einsatz eine Art Kernelement dar, die den axialen hohlen Durchlass auskleidet und umgibt. Das Bandmaterial ist spiralförmig um den versteifenden Einsatz gewickelt.

Gemäß einer weiteren Ausgestaltung der Erfindung kann das hintere Ende des versteifenden Einsatzes mit dem Bandmaterial verbunden sein. Dies verhindert ein Verrutschen des versteifenden Einsatzes und erhöht somit ebenfalls die Steifigkeit. Auch kann die Herstellung der erfindungsgemäßen Rolle dadurch vereinfacht werden. Die Verbindung kann beispielsweise eine leichte Haftung sein, die sich nach dem Abrollen der Bandage von allein oder durch leichten Zug lösen lässt.

Der versteifende Einsatz kann ein Flächengewicht zwischen 20 und 500 g/m² und vorzugsweise zwischen 60 bis 120 g/m² aufweisen. Dies ist abhängig von der erforderlichen Steifigkeit der Rolle und gleichzeitig abhängig von den Materialeigenschaften des Bandmaterials. Generell darf das Flächengewicht nur so groß gewählt werden, dass der versteifende Einsatz zusammen mit dem Bandmaterial spiralförmig zu einer Rolle wickelbar ist. Die genannten Bereiche für das Flächengewicht ermöglichen den Einsatz von diversen Standardmaterialien wie Papier und Folien, was zu einer Kostenreduzierung führt.

Der versteifende Einsatz kann eine Vielzahl an unterschiedlichen Materialien umfassen. Das Material kann ausgewählt sein aus der Gruppe bestehend aus Papier, Papiertuchmaterial, nicht gewebtes Material, mit einem Bindemittel behandeltes Papiertuchmaterial, mit einem Bindemittel behandeltes nicht gewebtes Material, Pappe, Kraftpapier, Polyurethan, Polyester oder synthetisches Polymer. Dies weist den Vorteil auf, dass leicht verfügbare und kostengünstige Materialien verwendet werden können.

Besonders bevorzugt ist der versteifende Einsatz aus Papier, das leicht und kostengünstig verfügbar ist und gut recycelt werden kann.

Gemäß einer bevorzugten Ausführungsform ist vorgesehen, dass der versteifende Einsatz eine Breite aufweist, die gleich einer Breite des Bandmaterials ist. Somit ist sichergestellt, dass die Rolle auf ihrer gesamten Breite durch den versteifenden Einsatz stabilisiert ist und sich das Bandmaterial gut von der Rolle abrollen lässt.

Vorzugsweise ist das Bandmaterial elastisch. Das Bandmaterial kann dabei in Längs- und/oder Querrichtung elastisch sein. Dabei kann insbesondere vorgesehen sein, dass das Bandmaterial ein Gewebe, Gewirke, Gestrick oder Vlies ist. Die Elastizität kann dabei durch die Einarbeitung von elastischen Fäden, wie beispielsweise hochgedrehten Baumwollfäden, als Spinn- oder Zwirnkreppfäden, durch texturierte Polyamid- oder Polyestergarne sowie Gummi- oder Polyurethanfäden bzw. Kombinationen hiervon hervorgerufen werden.

Der erfindungsgemäße axiale hohle Durchlass verläuft entlang der Mittelachse der Rolle, die im Wesentlichen als ein Zylinder ausgebildet ist. Vorzugsweise kann der axiale hohle Durchlass einen Durchmesser zwischen 0,5 cm und 5 cm und insbesondere 3 cm aufweisen. Dies ermöglicht einerseits eine stabile leicht aufwickelbare Rolle bereitzustellen. Andererseits kann die Rolle von einem Benutzer gut gehalten werden, indem er einen Finger der einen Hand in dem axialen hohlen Durchlass einführt und mit der anderen Hand die Bandage abrollt.

Gemäß einer bevorzugten Ausführungsform handelt es sich bei dem Bandmaterial um ein Gewebe, Gewirke, Gestricke oder Vlies. Erfindungsgemäß eignet sich die Rolle der Bandage, auch Binde genannt, für Fixier-, Kompressions-, Stütz- und Entlastungsverbände für medizinische Zwecke. Diese Verbände sind oft kohäsiv ausgebildet, so dass sie auf sich selbst haften und ohne weitere Fixiermittel angelegt werden können. Sie neigen daher dazu, ohne eine stabilisierende innere Hülse auf sich selbst zu verkleben und sich somit nicht bis zum Ende problemlos abrollen zu lassen.

Im Unterschied zu den mit adhäsiven Klebemassen beschichteten Verbandstoffen, die nahezu auf allen Gebrauchsgegenständen und Materialien haften und einen dauerhaften, aggressiven Tack besitzen, zeichnet sich der rein kohäsive Klebeeffekt dadurch aus, dass die Klebekräfte lediglich innerhalb der Klebemasse wirksam sind und den inneren Zusammenhang der Masse bewirken. Eine mit einer kohäsiven Klebemasse beschichtete Binde haftet demnach nur auf sich selbst, **d.h.** Lage auf Lage bzw. Bindentour auf Bindentour, aber nicht auf fremden Oberflächen wie **z.B.** Haut, Haaren oder Kleidungsstücken. Dieses kohäsive Klebe- oder Haftverhalten wurde bislang überwiegend durch Verwendung von aufeinander haftenden Oberflächenstrukturen, wie beispielsweise in WO 2007/131605 A1 beschrieben, oder durch Verwendung von kohäsiven Polymeren eingestellt. Zur Vermeidung von Allergien beim Nutzer der Binde handelt es sich bei dem kohäsiven Polymer vorzugsweise um ein Latex-freies synthetisches Polymer. Es ist auch möglich, die kohäsive Haftung durch eine Kombination einer auf sich selbst haftenden insbesondere gekreppten Oberflächenstruktur mit einer Imprägnierung mit einem haftenden synthetischen Polymer zu erreichen, wie dies beispielsweise bei der elastische Fixierbinden "Peha-haft" der Anmelderin der Fall ist.

Gemäß einer weiteren Ausführungsform ist vorgesehen, dass das Bandmaterial mit einer weiteren Substanz getränkt oder beschichtet ist. Die erfindungsgemäße Rolle ist in keiner Weise limitierend bezüglich des Substrats des Bandmaterials, der kohäsiven Klebemasse oder weiterer Substanzen, die eine Bandage umfassen kann.

Die Aufgabe wird des Weiteren durch ein Verfahren zum Herstellen von Rollen einer Bandage zum Anlegen an einen menschlichen oder tierischen Körper gelöst. Erfindungsgemäß ist vorgesehen, dass in einem ersten Schritt ein flächiges und kohäsiv ausgestaltetes Bandmaterial mit einem freistehenden Ende zugeführt wird. Entlang des freistehenden Endes ist ein Endabschnitt angeordnet. Zudem wird vor dem ersten Schritt, zeitgleich oder anschließend ein versteifender Einsatz zugeführt und in dem Endabschnitt des Bandmaterials positioniert. Der versteifende Einsatz wird auf dem Endabschnitt des Bandmaterials fixiert und in einem nächsten Schritt das Bandmaterial mit einer Vielzahl an Windungen spiralförmig aufgewickelt. So entsteht ein Langstück mit einem axialen hohlen Durchlass, wobei der axiale hohle Durchlass in Bezug auf das Langstück mittig angeordnet ist und sich entlang einer Querrichtung des Langstücks erstreckt. Der versteifende Einsatz umgibt wenigstens entlang einer vollständigen Windung den axialen hohlen Durchlass. Nachfolgend wird das Bandmaterial rechtwinklig in Bezug auf die Längsrichtung des Bandmaterials durchtrennt, um das Langstück von dem Vorrat an Bandmaterial, welches noch nicht erfindungsgemäß aufgewickelt ist, zu trennen. In einem letzten Schritt wird das Langstück in eine Anzahl an Rollen geschnitten.

Das erfindungsgemäße Verfahren weist den Vorteil auf, dass keine kostenintensive vorgefertigte Hülse bereitgestellt werden muss, sondern ein Versteifungsbereich im Wickelprozess der Rolle bzw. des Langstücks hergestellt wird. Dies führt nicht nur zu einer Kostenoptimierung durch die Verwendung des günstigen Materials für den versteifenden Einsatz, sondern auch zu einer Optimierung und Verkürzung des Herstellungsprozesses. Der versteifende Einsatz ersetzt somit auf eine günstige und effektive Weise die sonst übliche starre Hülse.

Vorteilhaft ist auch, dass die Bandagen nicht einzeln auf bereitgestellte starre Hülsen gewickelt werden müssen, sondern ein Langstück hergestellt werden kann, das anschließend in einzelne Rollen geschnitten wird. Somit ist die Breite der Bandage nicht von der Hülse abhängig und kann fertigungstechnisch einfach nach Bedarf angepasst werden. Das Bandmaterial sowie der versteifende Einsatz sind in nahezu jeder Breite und Länge verfügbar. Dies erlaubt zudem einen hohen Durchsatz.

Der versteifende Einsatz kann vorzugsweise lösbar im Endabschnitt fixiert werden.

Gemäß einer besonders bevorzugten Ausführungsform ist vorgesehen, dass der versteifende Einsatz mittels Unterdruck oder Sog im Endabschnitt fixiert wird. Hierzu wird durch das Bandmaterial hindurch der versteifende Einsatz angesaugt und somit temporär im Endabschnitt gehalten. Sobald der versteifende Einsatz wenigstens teilweise mit dem Bandmaterial eingewickelt ist, kann die Fixierung beendet werden, da durch das Aufwickeln der versteifende Einsatz sicher im Endabschnitt gehalten wird. Dies ermöglicht ein schnelles Herstellungsverfahren bei gleichzeitiger Einsparung von Rohstoffen wie zum Beispiel Klebstoff.

Zur temporären Stabilisierung des axialen hohlen Durchlasses bzw. um dem Durchlass eine bestimmte Form zu geben, kann vor dem spiralförmigen Aufwickeln zeitweise ein Kern auf dem versteifenden Einsatz angeordnet werden, wobei das Bandmaterial und der versteifende Einsatz um den Kern spiralförmig gewickelt werden. Der Kern wird vor dem Schneiden des Langstücks wieder entfernt und kann wiederverwendet werden.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden.

Es zeigen:
Figur 1 einen schematischen Schnitt durch eine Rolle mit sich überlappendem versteifenden Einsatz (nicht erfindungsgemäß),
Figur 2 eine weitere Anordnung des versteifenden Einsatzes in der Rolle gemäß Figur 1,
Figur 3 die Rolle aus Figur 1, jedoch mit einem den axialen hohlen Durchlass auskleidenden versteifenden Einsatz (nicht erfindungsgemäß),
Figur 4 eine schematische Ansicht des versteifenden Einsatzes im Endabschnitt des Bandmaterials,
Figur 5 eine weitere Anordnung des versteifenden Einsatzes aus Figur 4,
Figur 6 eine schematische seitliche Ansicht der Anordnung des versteifenden Einsatzes im Endabschnitt,
Figur 7 eine alternative Anordnung zu Figur 6,
Figur 8 eine perspektivische Ansicht der teilweise abgerollten Rolle und
Figur 9 eine schematische Darstellung des Herstellungsverfahrens.

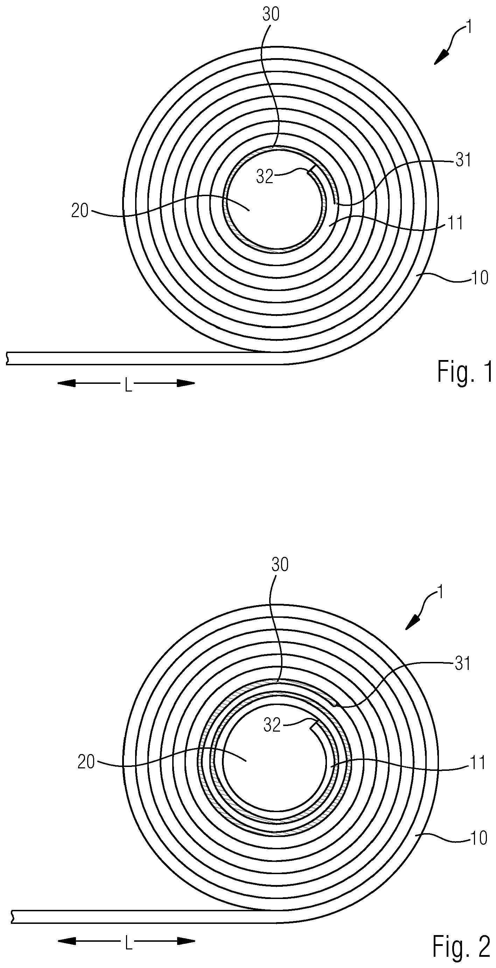

Figur 1 zeigt einen Schnitt in Längsrichtung L durch eine Rolle 1. Ein Bandmaterial 10 ist spiralförmig aufgewickelt, so dass mittig entlang der Achse der zylinderförmigen Rolle 1 ein axialer hohler Durchlass 20 gebildet ist. Dieser ist von einem versteifenden Einsatz 30 aus Papier ausgekleidet. Ausgekleidet bedeutet erfindungsgemäß, dass der versteifende Einsatz 30 die innere Windung bzw. Lage im hohlen axialen Durchlass 20 bildet und diesen ähnlich einer Hülse ausfüllt.

Überraschender Weise wurde festgestellt, dass als versteifender Einsatz 30 herkömmliches Papier mit einem Flächengewicht von 80 g/m² der Rolle 1 ausreichend Stabilität verleiht und ein Verkleben des freistehenden Endes des Bandmaterials 10 zuverlässig verhindert.

Der versteifende Einsatz 30 weist ein vorderes Ende 31 und ein hinteres Ende 32 auf, wobei das hintere Ende 32 am zum axialen hohlen Durchlass 20 orientierten freistehenden Ende des Bandmaterials 10 angeordnet ist. Der versteifende Einsatz 30 liegt mit seiner gesamten Fläche in einem Endabschnitt 11 des Bandmaterials 10 auf. Als Endabschnitt 11 wird der Bereich am freistehenden Ende des Bandmaterials 10 bezeichnet, der im Inneren der Rolle 1, also am axialen hohlen Durchlass 20, liegt.

Die Länge des versteifenden Einsatzes 30 ist so gewählt, dass sich das vordere Ende 31 und das hintere Ende 32 überlappen. Die beiden Enden 31 und 32 liegen in etwa übereinander und sind von einer Lage des Bandmaterials 10 getrennt.

Alternativ kann der versteifende Einsatz 30 auch erst die zweite Lage im axialen hohlen Durchlass 20 bilden, wobei die innere Lage durch das Bandmaterial 10 gebildet ist, wie in Figur 2 dargestellt. Der versteifende Einsatz 30 kleidet in diesem Ausführungsbeispiel den axialen hohlen Durchlass nicht aus, bildet aber dennoch einen Versteifungsbereich und verhindert ein Kollabieren des axialen hohlen Durchlasses 20. Die Länge des versteifenden Einsatzes 30 ist in Figur 2 derart eingerichtet, dass sich der versteifenden Einsatz 30 in der Längsrichtung L über zwei Windungen selbst überlappt, um einen spiralförmigen Versteifungsbereich zu bilden.

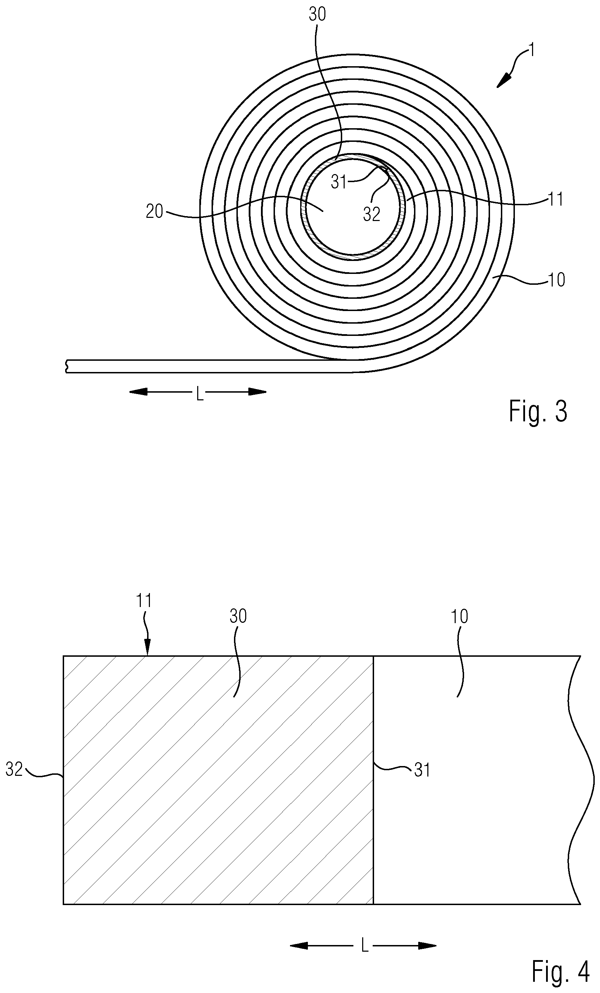

Figur 3 zeigt ebenfalls die Rolle 1 gemäß Figur 1, jedoch ist die Länge des versteifenden Einsatzes 30 so bemessen, dass er den axialen hohlen Durchlass 20 exakt entlang der ersten Windung auskleidet. Das vordere Ende 31 und das hintere Ende 32 kommen dabei auf Stoß aneinander zu liegen. Somit liegen die Stirnflächen am vorderen Ende 31 und am hinteren Ende 32 aneinander, ohne jedoch miteinander verbunden zu sein. Für diese Ausführung der Erfindung kommen bevorzugt Materialien mit höherem Flächengewicht für den versteifenden Einsatz 30 in Frage, beispielsweise in einem Bereich von 100 g/m² bis 200 g/m².

Figur 4 zeigt eine schematische Draufsicht auf den Endabschnitt 11 des Bandmaterials 10. Darauf angeordnet ist der versteifende Einsatz 30, wobei der versteifende Einsatz 30 in diesem Beispiel den Endabschnitt 11 vollständig bedeckt. Der versteifende Einsatz 30 liegt somit mit seiner gesamten Fläche im Endabschnitt 11 auf dem Bandmaterial 10 auf.

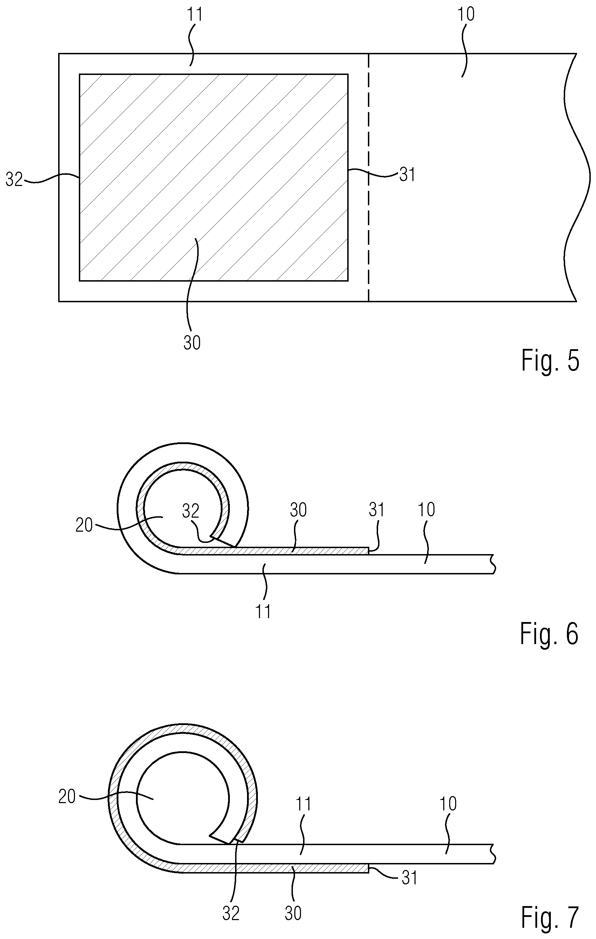

Alternativ kann der versteifende Einsatz 30 auch von den Kanten des Bandmaterials 10 geringfügig beabstandet sein, wie es in Figur 5 dargestellt ist. Dabei liegt der versteifende Einsatz 30 ebenfalls vollflächig im Bereich des Endabschnitts 11 auf dem Bandmaterial 10 auf. Dies kann in gewissen Maschinenkonfigurationen erforderlich sein, jedoch werden hierdurch die erfindungsgemäßen Vorteile nicht beeinträchtigt.

Die Figuren 6 und 7 zeigen einen schematischen Schnitt durch den Versteifungsbereich. Dieser verläuft um den axialen hohlen Durchlass 20, indem das Bandmaterial 10 spiralförmig wenigstens entlang einer Windung um die zentrale Achse der Rolle 1 aufgewickelt ist. Der versteifende Einsatz 30 ist in Figur 6 auf der nach innen, also hin zur Achse der Rolle 1, gerichteten Oberfläche des Bandmaterials 10 im Endabschnitt 11 angeordnet, so dass der versteifende Einsatz 30 den axialen hohlen Durchlass 20 vollständig auskleidet.

In dem Ausführungsbeispiel gemäß Figur 7 wurde im Herstellungsverfahren der versteifende Einsatz 30 auf der nach außen gerichteten Oberfläche des Bandmaterials 10 im Endabschnitt 11 angeordnet. Diese Oberfläche ist also von der Achse der Rolle 1 weggerichtet. Im spiralförmig aufgewickelten Zustand der Bandage kleidet der versteifende Einsatz 30 dann den axialen hohlen Durchlass 20 nicht aus. Die innere Lage entlang der ersten Windung ist von dem Bandmaterial 10 gebildet.

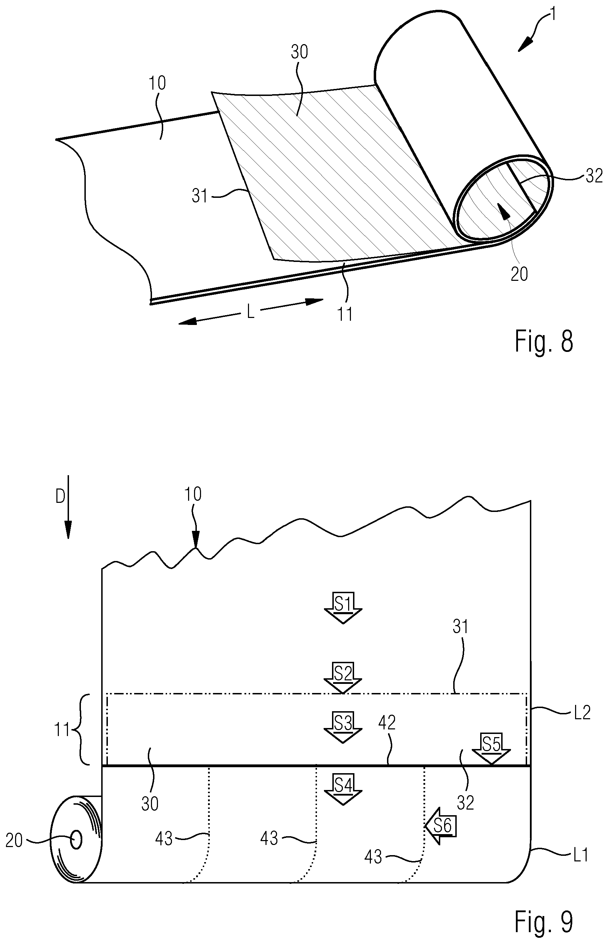

Figur 8 zeigt eine perspektivische Ansicht der Rolle 1 im Versteifungsbereich. Das Bandmaterial 10 ist bis auf 1,5 Windungen abgewickelt. Da der versteifende Einsatz 30 nicht mit dem Bandmaterial 10 verbunden, beispielsweise verklebt ist, steht das vordere Ende 31 leicht vom Endabschnitt ab.

Zum Anlegen der Bandage am Körper kann der Benutzer mit den Fingern einer Hand die Rolle 1 im axialen hohlen Durchlass 20 greifen und mit der anderen Hand an dem freistehenden Ende des Bandmaterials 10 ziehen. Dadurch, dass im Endabschnitt 11 der versteifende Einsatz 30 aufliegt, lässt sich das Bandmaterial 10 vollständig von der Rolle 1 abrollen, ohne dass das im Inneren der Rolle 1 liegende Stück des Bandmaterials 10 mit sich selbst verklebt und mühsam wieder auseinandergezogen werden muss. Sobald die Rolle 1 vollständig abgerollt ist, kann der versteifende Einsatz 30 abgenommen werden oder löst sich bereits von alleine, da er im Endabschnitt 11 nicht mehr fixiert ist.

Figur 9 zeigt schematisch das Herstellungsverfahren der erfindungsgemäßen Rolle **1.** Ein Langstück L1 ist bereits erfindungsgemäß gewickelt worden und wird im Schritt S5 von dem übrigen Bandmaterial 10 getrennt. Für ein zweites nachfolgendes Langstück L2 ist das Bandmaterial 10, welches kohäsiv ausgestaltet ist, mit einem Ende, das nach dem Abtrennen des ersten Langstücks L1 entlang des Schnitts 42 freisteht in der Maschine eingelegt. An dem freistehenden Ende befindet sich der Endabschnitt 11. Nachfolgend (S2) wird der bereits auf seine Länge gekürzte versteifende Einsatz 30 im Endabschnitt 11 des Bandmaterials 10 aufgelegt. Gemäß Figur 9 schließt das hintere Ende 32 des versteifenden Einsatzes 30 mit der Kante des freistehenden Endes des Bandmaterials 10 ab und ist auf der später nach innen gerichteten Oberfläche des Bandmaterials 10 angeordnet. In einem dritten Schritt (S3) wird im Endabschnitt 11 durch das Bandmaterial 10 hindurch ein Sog angelegt, mit dem der versteifende Einsatz 3 auf dem Endabschnitt 11 des Bandmaterials 10 temporär fixiert wird. Anschließend wird beginnend am Endabschnitt 11 das Bandmaterial 10 mit einer Vielzahl an Windungen spiralförmig aufgewickelt (S4), so dass die Langstücke mit einem axialen hohlen Durchlass 20 ausgebildet werden. Der axiale hohle Durchlass 20 ist mittig um die Achse der Langstücke (wie für das Langstück L1 dargestellt) angeordnet und erstreckt sich quer zur Längsrichtung des Bandmaterials 10. Somit kommt der versteifende Einsatz 30 wenigstens entlang einer vollständigen Windung im axialen hohlen Durchlass 20 zu liegen und kleidet den axialen hohlen Durchlass 20 aus.

Das fertig gewickelte Langstücks L1 wird durch Durchtrennen des Bandmaterial 10 entlang eines quer verlaufenden Schnitts 42 von dem Vorrat an Bandmaterial 10 getrennt (S5). Das Herstellungsverfahren kann nun erneut für die Herstellung eines weiteren Langstücks beginnen.

In einem letzten Schritt wird das Langstück L1 in die vorbestimmte Anzahl an Rollen 1 geschnitten, indem das Langstück L1 entlang der Schneidlinien 43 längs durchtrennt wird (S6).

## Patentansprüche

1. Rolle (1) einer medizinischen Bandage zum Anlegen an einen menschlichen oder tierischen Körper, umfassend ein mit einer Vielzahl an Windungen spiralförmig gewickeltes flächiges und kohäsiv ausgestaltetes Bandmaterial (10), wobei das Bandmaterial (10) einen Endabschnitt (11) aufweist und derart in einer Längsrichtung (L) gewickelt ist, dass die Rolle (1) mit einem axialen hohlen Durchlass (20) gebildet ist, wobei der axiale hohle Durchlass (20) in Bezug auf die Rolle (1) mittig angeordnet ist und sich entlang einer Querrichtung des Bandmaterials (10) erstreckt und wobei der Endabschnitt (11) im Inneren der Rolle (1) am axialen hohlen Durchlass (20) liegt, **dadurch gekennzeichnet, dass** ein versteifender Einsatz (30) aus einem flächigen Material mit einem vorderen Ende (31) und einem hinteren Ende (32) vorgesehen ist, wobei der versteifende Einsatz (30) im Endabschnitt (11) auf dem Bandmaterial (10) aufliegt, insbesondere über seine gesamte Fläche, und der versteifende Einsatz (30) wenigstens entlang einer vollständigen Windung den axialen hohlen Durchlass (20) umgibt, wobei sich der versteifende Einsatz (30) in der Längsrichtung (L) über wenigstens zwei Windungen selbst überlappt, um einen spiralförmig ausgebildeten Versteifungsbereich zu bilden.

2. Rolle (1) nach Anspruch 1, **dadurch gekennzeichnet, dass** der versteifende Einsatz (30) eine Länge aufweist, die so gewählt ist, dass sich das vordere Ende (31) und das hintere Ende (32) des versteifenden Einsatzes (30) einander überlappen.

3. Rolle (1) nach einem der vorhergehenden Ansprüche, **dadurch gekennzeichnet, dass** der der versteifende Einsatz (30) ein Flächengewicht zwischen 20 und 500 g/m² und vorzugsweise zwischen 60 bis 120 g/m² aufweist.

4. Rolle (1) nach einem der vorhergehenden Ansprüche, **dadurch gekennzeichnet, dass** der versteifende Einsatz (30) eine Breite aufweist, die gleich der Breite des Bandmaterials (10) ist.

5. Rolle (1) nach einem der vorhergehenden Ansprüche, **dadurch gekennzeichnet, dass** der axiale hohle Durchlass (20) einen Durchmesser zwischen 0,5 cm und 5 cm und insbesondere 3 cm aufweist.

6. Rolle (1) nach einem der vorhergehenden Ansprüche, **dadurch gekennzeichnet, dass** der versteifende Einsatz (30) ein Material ausgewählt aus der Gruppe bestehend aus Papiertuchmaterial, nicht gewebtes Material, mit einem Bindemittel behandeltes Papiertuchmaterial, mit einem Bindemittel behandeltes nicht gewebtes Material, Pappe, Kraftpapier, Polyurethan, Polyester oder synthetisches Polymer, besonders bevorzugt Papier oder Folie umfasst.

7. Rolle (1) nach einem der vorhergehenden Ansprüche, **dadurch gekennzeichnet, dass** das Bandmaterial (10) ein Gewebe, Gewirke, Gestrick oder Vlies ist.

8. Rolle (1) nach einem der vorhergehenden Ansprüche, **dadurch gekennzeichnet, dass** das Bandmaterial (10) mit einer vorzugsweise Latex-freien kohäsiven Klebemasse beschichtet ist und/oder adhäsive Oberflächenstrukturen trägt, so dass das Bandmaterial auf sich selbst haftet.

9. Rolle (1) nach einem der vorhergehenden Ansprüche, **dadurch gekennzeichnet, dass** das Bandmaterial (10) mit einer weiteren Substanz getränkt ist.

10. Verfahren zum Herstellen von Rollen (1) einer Bandage gemäß den Ansprüche 1-9 zum Anlegen an einen menschlichen oder tierischen Körper umfassend die folgenden Schritte:
- Zuführen (S1) eines flächigen und kohäsiv ausgestalteten Bandmaterials (10) mit einem freistehenden Ende, wobei entlang des freistehenden Endes ein Endabschnitt (11) angeordnet ist,
- Zuführen (S2) eines versteifenden Einsatzes (30) und Positionieren in dem Endabschnitt (11) des Bandmaterials (10),
- Fixieren (S3) des versteifenden Einsatzes (30) auf dem Endabschnitt (11) des Bandmaterials (10),
- spiralförmiges Aufwickeln (S4) des Bandmaterials (10) mit einer Vielzahl an Windungen beginnend am freistehenden Ende, so dass ein Langstück (L1) mit einem axialen hohlen Durchlass (20) ausgebildet wird, wobei der axiale hohle Durchlass (20) in Bezug auf das Langstück (L1) mittig angeordnet ist und sich entlang einer Querrichtung des Langstücks (L1) erstreckt, wobei der versteifende Einsatz (30) wenigstens entlang einer vollständigen Windung den axialen hohlen Durchlass (20) umgibt und sich der versteifende Einsatz (30) in einer Längsrichtung (L) über wenigstens zwei Windungen selbst überlappt, um einen spiralförmig ausgebildeten Versteifungsbereich zu bilden,
- Durchtrennen (S5) des Bandmaterials (10) rechtwinklig zur Längsrichtung des Bandmaterials (10),
- Schneiden (S6) des Langstücks (L1) in eine Anzahl an Rollen (1).

11. Verfahren nach Anspruch 10, **dadurch gekennzeichnet, dass** der versteifende Einsatz (30) lösbar fixiert wird.

12. Verfahren nach Anspruch 10 oder 11, **dadurch gekennzeichnet, dass** der versteifende Einsatz (30) mittels Unterdruck oder Sog fixiert wird.

13. Verfahren nach einem der Ansprüche 10 bis 12, **dadurch gekennzeichnet, dass** vor dem spiralförmigen Aufwickeln (S4) zeitweise ein Kern auf dem versteifenden Einsatz (30) angeordnet ist, wobei das Bandmaterial (10) und der versteifende Einsatz (30) um den Kern spiralförmig gewickelt werden und der Kern den vorbestimmten axialen hohlen Durchlass (20) stützt.

## Claims

1. Roll (1) of a medical bandage for applying to a human or animal body, comprising a planar and cohesive strip material (10) wound in a spiral shape with multiple windings, wherein the strip material (10) has an end portion (11) and is wound in a longitudinal direction (L) in such a way that the roll (1) is formed with an axial hollow passage (20), wherein the axial hollow passage (20) is located centrally with respect to the roll (1) and extends along a transverse direction of the strip material (10), and wherein the end portion (11) in the interior of the roll (1) lies on the axial hollow passage (20), **characterized in that** a stiffening insert (30) made of a planar material with a front end (31) and a rear end (32) is provided, wherein the stiffening insert (30) lies on the strip material (10) in the end portion (11), in particular over its entire surface area, and the stiffening insert (30) surrounds the axial hollow passage (20) at least along one complete winding, wherein the stiffening insert (30) overlaps itself in the longitudinal direction (L) over at least two windings, in order to form a spiral-shaped stiffening region.

2. Roll (1) according to Claim 1, **characterized in that** the stiffening insert (30) has a length selected such that the front end (31) and the rear end (32) of the stiffening insert (30) overlap each other.

3. Roll (1) according to either of the preceding claims, **characterized in that** the stiffening insert (30) has a basis weight of between 20 and 500 g/m² and preferably of between 60 and 120 g/m².

4. Roll (1) according to any one of the preceding claims, **characterized in that** the stiffening insert (30) has a width which is equal to the width of the strip material (10).

5. Roll (1) according to any one of the preceding claims, **characterized in that** the axial hollow passage (20) has a diameter of between 0.5 cm and 5 cm, in particular 3 cm.

6. Roll (1) according to any one of the preceding claims, **characterized in that** the stiffening insert (30) comprises a material selected from the group consisting of paper towel material, nonwoven material, paper towel material treated with a binder, nonwoven material treated with a binder, cardboard, kraft paper, polyurethane, polyester or synthetic polymer, particularly preferably paper or film.

7. Roll (1) according to any one of the preceding claims, **characterized in that** the strip material (10) is a woven fabric, knitted fabric, crocheted fabric or nonwoven fabric.

8. Roll (1) according to any one of the preceding claims, **characterized in that** the strip material (10) is coated with a preferably latex-free cohesive adhesive composition and/or bears adhesive surface structures, such that the strip material adheres to itself.

9. Roll (1) according to any one of the preceding claims, **characterized in that** the strip material (10) is impregnated with a further substance.

10. Method for producing rolls (1) of a bandage according to Claims 1-9 for applying to a human or animal body, the method comprising the following steps:
- feeding (S1) a planar and cohesive strip material (10) having a free-standing end, wherein an end portion (11) is arranged along the free-standing end,
- feeding (S2) a stiffening insert (30) and positioning same in the end portion (11) of the strip material (10),
- fixing (S3) the stiffening insert (30) on the end portion (11) of the strip material (10),
- spirally winding (S4) the strip material (10) with multiple windings beginning at the free-standing end, such that an elongate piece (L1) with an axial hollow passage (20) is formed, wherein the axial hollow passage (20) is located centrally with respect to the elongate piece (L1) and extends along a transverse direction of the elongate piece (L1), wherein the stiffening insert (30) surrounds the axial hollow passage (20) at least along one complete winding, and the stiffening insert (30) overlaps itself in a longitudinal direction (L) over at least two windings, in order to form a spiral-shaped stiffening region,
- severing (S5) the strip material (10) at right angles to the longitudinal direction of the strip material (10),
- cutting (S6) the elongate piece (L1) into a number of rolls (1).

11. Method according to Claim 10, **characterized in that** the stiffening insert (30) is fixed releasably.

12. Method according to Claim 10 or 11, **characterized in that** the stiffening insert (30) is fixed by negative pressure or suction.

13. Method according to any one of Claims 10 to 12, **characterized in that**, before the spiral winding (S4), a core is temporarily arranged on the stiffening insert (30), wherein the strip material (10) and the stiffening insert (30) are wound spirally around the core, and the core supports the predetermined axial hollow passage (20).

## Revendications

1. Rouleau (1) d'un bandage médical destiné à être appliqué sur un corps humain ou animal, comprenant un matériau de bande (10) plat et à caractère cohésif, enroulé en spirale avec une pluralité de spires, le matériau de bande (10) présentant une section d'extrémité (11) et étant enroulé dans une direction longitudinale (L) de telle sorte que le rouleau (1) est formé avec un passage creux axial (20), le passage creux axial (20) étant disposé au centre par rapport au rouleau (1) et s'étendant selon une direction transversale du matériau de bande (10), et la section d'extrémité (11) étant située à l'intérieur du rouleau (1) au niveau du passage creux axial (20), **caractérisé en ce qu'**un insert de rigidification (30) en un matériau plat, présentant une extrémité avant (31) et une extrémité arrière (32), est prévu, l'insert de rigidification (30) reposant sur le matériau de bande (10) dans la section d'extrémité (11), notamment sur toute sa surface, et l'insert de rigidification (30) entourant le passage creux axial (20) sur au moins une spire complète, l'insert de rigidification (30) se chevauchant lui-même dans la direction longitudinale (L) sur au moins deux spires afin de former une zone de rigidification réalisée en spirale.

2. Rouleau (1) selon la revendication 1, **caractérisé en ce que** l'insert de rigidification (30) présente une longueur choisie de telle sorte que l'extrémité avant (31) et l'extrémité arrière (32) de l'insert de rigidification (30) se chevauchent l'une l'autre.

3. Rouleau (1) selon l'une quelconque des revendications précédentes, **caractérisé en ce que** l'insert de rigidification (30) présente une masse surfacique comprise entre 20 et 500 g/m² et, de préférence, entre 60 et 120 g/m².

4. Rouleau (1) selon l'une quelconque des revendications précédentes, **caractérisé en ce que** l'insert de rigidification (30) présente une largeur égale à la largeur du matériau de bande (10).

5. Rouleau (1) selon l'une quelconque des revendications précédentes, **caractérisé en ce que** le passage creux axial (20) présente un diamètre compris entre 0,5 cm et 5 cm et, en particulier, de 3 cm.

6. Rouleau (1) selon l'une quelconque des revendications précédentes, **caractérisé en ce que** l'insert de rigidification (30) comprend un matériau sélectionné dans le groupe constitué par un matériau en papier tissu, un matériau non tissé, un matériau en papier tissu traité avec un liant, un matériau non tissé traité avec un liant, du carton, du papier kraft, du polyuréthane, du polyester ou un polymère synthétique, de manière tout particulièrement préférée du papier ou un film.

7. Rouleau (1) selon l'une quelconque des revendications précédentes, **caractérisé en ce que** le matériau de bande (10) est un tissu, une étoffe tricotée, une étoffe à mailles jetées ou un non-tissé.

8. Rouleau (1) selon l'une quelconque des revendications précédentes, **caractérisé en ce que** le matériau de bande (10) est revêtu d'une masse adhésive cohésive, de préférence exempte de latex, et/ou porte des structures de surface adhésives, de sorte que le matériau de bande adhère à lui-même.

9. Rouleau (1) selon l'une quelconque des revendications précédentes, **caractérisé en ce que** le matériau de bande (10) est imprégné d'une substance supplémentaire.

10. Procédé de fabrication de rouleaux (1) d'un bandage selon les revendications 1 à 9 destiné à être appliqué sur un corps humain ou animal, comprenant les étapes suivantes :
- l'acheminement (S1) d'un matériau de bande (10) plat et à caractère cohésif présentant une extrémité libre, une section d'extrémité (11) étant disposée le long de l'extrémité libre,
- l'acheminement (S2) d'un insert de rigidification (30) et le positionnement de celui-ci dans la section d'extrémité (11) du matériau de bande (10),
- la fixation (S3) de l'insert de rigidification (30) sur la section d'extrémité (11) du matériau de bande (10),
- l'enroulement en spirale (S4) du matériau de bande (10) avec une pluralité de spires en commençant à l'extrémité libre, de telle sorte qu'un long tronçon (L1) est formé avec un passage creux axial (20), le passage creux axial (20) étant disposé au centre par rapport au long tronçon (L1) et s'étendant selon une direction transversale du long tronçon (L1), l'insert de rigidification (30) entourant le passage creux axial (20) sur au moins une spire complète et l'insert de rigidification (30) se chevauchant lui-même dans une direction longitudinale (L) sur au moins deux spires afin de former une zone de rigidification réalisée en spirale,
- le sectionnement (S5) du matériau de bande (10) perpendiculairement à la direction longitudinale du matériau de bande (10),
- la découpe (S6) du long tronçon (L1) en un nombre de rouleaux (1).

11. Procédé selon la revendication 10, **caractérisé en ce que** l'insert de rigidification (30) est fixé de manière amovible.

12. Procédé selon la revendication 10 ou 11, **caractérisé en ce que** l'insert de rigidification (30) est fixé au moyen d'une dépression ou d'une aspiration.

13. Procédé selon l'une quelconque des revendications 10 à 12, **caractérisé en ce qu'**avant l'enroulement en spirale (S4), un noyau est disposé temporairement sur l'insert de rigidification (30), le matériau de bande (10) et l'insert de rigidification (30) étant enroulés en spirale autour du noyau, et le noyau soutenant le passage creux axial (20) prédéterminé.
